# EUROPEAN PATENT APPLICATION

(11) **EP 1 486 570 A1**
(43) Date of publication of application: **15.12.2004**
(21) Application number: 04013414.0
(22) Date of filing: 07.06.2004
(51) Int. Cl.: C12P 13/06, C12P 13/08, C12P 13/14

(54) **Method for producing L-amino acid**

(30) Priority: 11.06.2003 JP 2003166654; 26.03.2004 JP 2004091708
(71) Applicant: Ajinomoto Co., Inc., Tokyo (JP)
(72) Inventor: Imaizumi, Akira, Kawasaki-shi, Kanagawa (JP); Shakulov, Rustem Saidovich, 1st Dorozhny pr., 1, Moscow 113545 (RU); Briukhanov, Andrey Leonidovich, 1st Dorozhny pr., 1, Moscow 113545 (RU)
(74) Representative: Strehl Schübel-Hopf & Partner

(57) **Abstract**

An L-amino acid is produced by culturing a bacterium having an ability to produce an L-amino acid in a medium to allow accumulation of the L-amino acid in a culture and by collecting the L-amino acid from the culture, the bacterium being modified so that intracellular ppGpp synthesis ability is increased.

SEQUENCE LISTING

<110>  Ajinomoto Co., Inc.

<120>  Method for producing L-amino acid

<130>  EPA-63482

<150>  JP 2003-166654
<151>  2003-06-11

<150>  JP 2004-91708
<151>  2004-03-26

<160>  22

<170>  PatentIn version 3.0

<210>  1
<211>  28
<212>  DNA
<213>  artificial

<220>
<223>  primer sucA1

<400>  1
gcgaattcct gcccctgaca ctaagaca                                          28

<210>  2
<211>  20
<212>  DNA
<213>  artificial

<220>
<223>  primer sucA2

<400>  2
cgaggtaacg ttcaagacct                                                   20

<210>  3
<211>  40
<212>  DNA
<213>  artificial

<220>
<223> primer sucA3

<400> 3
aggtcttgaa cgttacctcg atccataacg ggcagggcgc 40

<210> 4
<211> 28
<212> DNA
<213> artificial

<220>
<223> primer sucA4

<400> 4
gcgaattccc actttgtcag tttcgatt 28

<210> 5
<211> 28
<212> DNA
<213> artificial

<220>
<223> primer relA1

<400> 5
gcgaattctt gaactggtac aggcaacc 28

<210> 6
<211> 42
<212> DNA
<213> artificial

<220>
<223> primer relA2

<400> 6
tgtttaagtt tagtggatgg gtgcgtctgt tgcagacaat ac 42

<210> 7
<211> 41
<212> DNA
<213> artificial

<220>

<223>  primer relA3

<400>  7
cccatccact aaacttaaac atagcgacac caaacagcaa c                41

<210>  8
<211>  29
<212>  DNA
<213>  artificial

<220>
<223>  primer relA4

<400>  8
gcgaattcaa gcacttcact actgttttc                29

<210>  9
<211>  25
<212>  DNA
<213>  artificial

<220>
<223>  primer relA5

<400>  9
tttaagcttg cgcgactggc gatgc                25

<210>  10
<211>  24
<212>  DNA
<213>  artificial

<220>
<223>  primer relA6

<400>  10
tttctagat ccgcaccgcc ggtg                24

<210>  11
<211>  28
<212>  DNA
<213>  artificial

<220>
<223>  primer relA7

<400> 11
ttttctagat aatttcaatc tggtcgcc 28

<210> 12
<211> 28
<212> DNA
<213> artificial

<220>
<223> primer relA8

<400> 12
gcgaattcta cgcactggct caataatt 28

<210> 13
<211> 28
<212> DNA
<213> artificial

<220>
<223> primer relA9

<400> 13
gcaagctttg tgacgtttta tcacgaaa 28

<210> 14
<211> 31
<212> DNA
<213> artificial

<220>
<223> primer relA10

<400> 14
gcgaattcta gataatttca atctggtcgc c 31

<210> 15
<211> 29
<212> DNA
<213> artificial

<220>
<223> primer spoT1

<400> 15
gcgaattccg cggagtatct ttattttac 29

<210> 16
<211> 39
<212> DNA
<213> artificial

<220>
<223> primer spoT2

<400> 16
cccatccact aaacttaaac agctttcaaa cagatacaa 39

<210> 17
<211> 39
<212> DNA
<213> artificial

<220>
<223> primer spoT3

<400> 17
tgtttaagtt tagtggatgg gacccgaaac cgaaattaa 39

<210> 18
<211> 28
<212> DNA
<213> artificial

<220>
<223> primer spoT4

<400> 18
gcgaattcta aagaatgagg gctgaggc 28

※<210> 19
<211> 2235
<212> DNA
<213> Escherichia coli

<220>
<221> CDS
<222> (1)..(2235)

<400> 19
atg gtt gcg gta aga agt gca cat atc aat aag gct ggt gaa ttt gat      48
Met Val Ala Val Arg Ser Ala His Ile Asn Lys Ala Gly Glu Phe Asp
1               5                   10                  15
ccg gaa aaa tgg atc gca agt ctg ggt att acc agc cag aag tcg tgt      96
Pro Glu Lys Trp Ile Ala Ser Leu Gly Ile Thr Ser Gln Lys Ser Cys
            20                  25                  30
gag tgc tta gcc gaa acc tgg gcg tat tgt ctg caa cag acg cag ggg      144
Glu Cys Leu Ala Glu Thr Trp Ala Tyr Cys Leu Gln Gln Thr Gln Gly
        35                  40                  45
cat ccg gat gcc agt ctg tta ttg tgg cgt ggt gtt gag atg gtg gag      192
His Pro Asp Ala Ser Leu Leu Leu Trp Arg Gly Val Glu Met Val Glu
    50                  55                  60
atc ctc tcg aca tta agt atg gac att gac acg ctg cgg gcg gcg ctg      240
Ile Leu Ser Thr Leu Ser Met Asp Ile Asp Thr Leu Arg Ala Ala Leu
65                  70                  75                  80
ctt ttc cct ctg gcg gat gcc aac gta gtc agc gaa gat gtg ctg cgt      288
Leu Phe Pro Leu Ala Asp Ala Asn Val Val Ser Glu Asp Val Leu Arg
                85                  90                  95
gag agc gtc ggt aag tcg gtc gtt aac ctt att cac ggc gtg cgt gat      336
Glu Ser Val Gly Lys Ser Val Val Asn Leu Ile His Gly Val Arg Asp
            100                 105                 110
atg gcg gcg atc cgc cag ctg aaa gcg acg cac act gat tct gtt tcc      384
Met Ala Ala Ile Arg Gln Leu Lys Ala Thr His Thr Asp Ser Val Ser
        115                 120                 125
tcc gaa cag gtc gat aac gtt cgc cgg atg tta ttg gcg atg gtc gat      432
Ser Glu Gln Val Asp Asn Val Arg Arg Met Leu Leu Ala Met Val Asp
    130                 135                 140
gat ttt cgc tgc gta gtc atc aaa ctg gcg gag cgt att gct cat ctg      480
Asp Phe Arg Cys Val Val Ile Lys Leu Ala Glu Arg Ile Ala His Leu
145                 150                 155                 160
cgc gaa gta aaa gat gcg ccg gaa gat gaa cgt gta ctg gcg gca aaa      528
Arg Glu Val Lys Asp Ala Pro Glu Asp Glu Arg Val Leu Ala Ala Lys
                165                 170                 175
gag tgt acc aac atc tac gca ccg ctg gct aac cgt ctc gga atc gga      576
Glu Cys Thr Asn Ile Tyr Ala Pro Leu Ala Asn Arg Leu Gly Ile Gly
            180                 185                 190
caa ctg aaa tgg gaa ctg gaa gat tac tgc ttc cgt tac ctc cat cca      624
Gln Leu Lys Trp Glu Leu Glu Asp Tyr Cys Phe Arg Tyr Leu His Pro
        195                 200                 205
acc gaa tac aaa cga att gcc aaa ctg ctg cat gaa cgg cgt ctc gac      672
Thr Glu Tyr Lys Arg Ile Ala Lys Leu Leu His Glu Arg Arg Leu Asp
    210                 215                 220
cgc gaa cac tac atc gaa gag ttc gtt ggt cat ctg cgc gct gag atg      720
Arg Glu His Tyr Ile Glu Glu Phe Val Gly His Leu Arg Ala Glu Met

225                 230                 235                 240
aaa gct gaa ggc gtt aaa gcg gaa gtg tat ggt cgt ccg aaa cac atc      768
Lys Ala Glu Gly Val Lys Ala Glu Val Tyr Gly Arg Pro Lys His Ile
                245                 250                 255
tac agc atc tgg cgt aaa atg cag aaa aag aac ctc gcc ttt gat gag      816
Tyr Ser Ile Trp Arg Lys Met Gln Lys Lys Asn Leu Ala Phe Asp Glu
            260                 265                 270
ctg ttt gat gtg cgt gcg gta cgt att gtc gcc gag cgt tta cag gat      864
Leu Phe Asp Val Arg Ala Val Arg Ile Val Ala Glu Arg Leu Gln Asp
        275                 280                 285
tgc tat gcc gca ctg ggg ata gtg cac act cac tat cgc cac ctg ccg      912
Cys Tyr Ala Ala Leu Gly Ile Val His Thr His Tyr Arg His Leu Pro
    290                 295                 300
gat gag ttt gac gat tac gtc gct aac ccg aaa cca aac ggt tat cag      960
Asp Glu Phe Asp Asp Tyr Val Ala Asn Pro Lys Pro Asn Gly Tyr Gln
305                 310                 315                 320
tct att cat acc gtg gtt ctg ggg ccg ggt gga aaa acc gtt gag atc      1008
Ser Ile His Thr Val Val Leu Gly Pro Gly Gly Lys Thr Val Glu Ile
                325                 330                 335
caa atc cgc acc aaa cag atg cat gaa gat gca gag ttg ggt gtt gct      1056
Gln Ile Arg Thr Lys Gln Met His Glu Asp Ala Glu Leu Gly Val Ala
            340                 345                 350
gcg cac tgg aaa tat aaa gag ggc gcg gct gct ggc ggc gca cgt tcg      1104
Ala His Trp Lys Tyr Lys Glu Gly Ala Ala Ala Gly Gly Ala Arg Ser
        355                 360                 365
gga cat gaa gac cgg att gcc tgg ctg cgt aaa ctg att gcg tgg cag      1152
Gly His Glu Asp Arg Ile Ala Trp Leu Arg Lys Leu Ile Ala Trp Gln
    370                 375                 380
gaa gag atg gct gat tcc ggc gaa atg ctc gac gaa gta cgt agt cag      1200
Glu Glu Met Ala Asp Ser Gly Glu Met Leu Asp Glu Val Arg Ser Gln
385                 390                 395                 400
gtc ttt gac gac cgg gtg tac gtc ttt acg ccg aaa ggt gat gtc gtt      1248
Val Phe Asp Asp Arg Val Tyr Val Phe Thr Pro Lys Gly Asp Val Val
                405                 410                 415
gat ttg cct gcg gga tca acg ccg ctg gac ttc gct tac cac atc cac      1296
Asp Leu Pro Ala Gly Ser Thr Pro Leu Asp Phe Ala Tyr His Ile His
            420                 425                 430
agt gat gtc gga cac cgc tgc atc ggg gca aaa att ggc ggg cgc att      1344
Ser Asp Val Gly His Arg Cys Ile Gly Ala Lys Ile Gly Gly Arg Ile
        435                 440                 445
gtg ccg ttc acc tac cag ctg cag atg ggc gac cag att gaa att atc      1392
Val Pro Phe Thr Tyr Gln Leu Gln Met Gly Asp Gln Ile Glu Ile Ile
    450                 455                 460
acc cag aaa cag ccg aac ccc agc cgt gac tgg tta aac cca aac ctc      1440
Thr Gln Lys Gln Pro Asn Pro Ser Arg Asp Trp Leu Asn Pro Asn Leu

465                 470                 475                 480
ggt tac gtc aca acc agc cgt ggg cgt tcg aaa att cac gcc tgg ttc     1488
Gly Tyr Val Thr Thr Ser Arg Gly Arg Ser Lys Ile His Ala Trp Phe
                485                 490                 495
cgt aaa cag gac cgt gac aaa aac att ctg gct ggg cgg caa atc ctt     1536
Arg Lys Gln Asp Arg Asp Lys Asn Ile Leu Ala Gly Arg Gln Ile Leu
            500                 505                 510
gac gac gag ctg gaa cat ctg ggg atc agc ctg aaa gaa gca gaa aaa     1584
Asp Asp Glu Leu Glu His Leu Gly Ile Ser Leu Lys Glu Ala Glu Lys
        515                 520                 525
cat ctg ctg ccg cgt tac aac ttc aat gat gtc gac gag ttg ctg gcg     1632
His Leu Leu Pro Arg Tyr Asn Phe Asn Asp Val Asp Glu Leu Leu Ala
    530                 535                 540
gcg att ggt ggc ggg gat atc cgt ctc aat cag atg gtg aac ttc ctg     1680
Ala Ile Gly Gly Gly Asp Ile Arg Leu Asn Gln Met Val Asn Phe Leu
545                 550                 555                 560
caa tcg caa ttt aat aag ccg agt gcc gaa gag cag gac gcc gcc gcg     1728
Gln Ser Gln Phe Asn Lys Pro Ser Ala Glu Glu Gln Asp Ala Ala Ala
                565                 570                 575
ctg aag caa ctt cag caa aaa agc tac acg ccg caa aac cgc agt aaa     1776
Leu Lys Gln Leu Gln Gln Lys Ser Tyr Thr Pro Gln Asn Arg Ser Lys
            580                 585                 590
gat aac ggt cgc gtg gta gtc gaa ggt gtt ggc aac ctg atg cac cac     1824
Asp Asn Gly Arg Val Val Val Glu Gly Val Gly Asn Leu Met His His
        595                 600                 605
atc gcg cgc tgc tgc cag ccg att cct gga gat gag att gtc ggc ttc     1872
Ile Ala Arg Cys Cys Gln Pro Ile Pro Gly Asp Glu Ile Val Gly Phe
    610                 615                 620
att acc cag ggg cgc ggt att tca gta cac cgc gcc gat tgc gaa caa     1920
Ile Thr Gln Gly Arg Gly Ile Ser Val His Arg Ala Asp Cys Glu Gln
625                 630                 635                 640
ctg gcg gaa ctg cgc tcc cat gcg cca gaa cgc att gtt gac gcg gta     1968
Leu Ala Glu Leu Arg Ser His Ala Pro Glu Arg Ile Val Asp Ala Val
                645                 650                 655
tgg ggt gag agc tac tcc gcc gga tat tcg ctg gtg gtc cgc gtg gta     2016
Trp Gly Glu Ser Tyr Ser Ala Gly Tyr Ser Leu Val Val Arg Val Val
            660                 665                 670
gct aat gat cgt agt ggg ttg tta cgt gat atc acg acc att ctc gcc     2064
Ala Asn Asp Arg Ser Gly Leu Leu Arg Asp Ile Thr Thr Ile Leu Ala
        675                 680                 685
aac gag aag gtg aac gtg ctt ggc gtt gcc agc cgt agc gac acc aaa     2112
Asn Glu Lys Val Asn Val Leu Gly Val Ala Ser Arg Ser Asp Thr Lys
    690                 695                 700
cag caa ctg gcg acc atc gac atg acc att gag att tac aac ctg caa     2160
Gln Gln Leu Ala Thr Ile Asp Met Thr Ile Glu Ile Tyr Asn Leu Gln

705                 710                 715                 720
gtg ctg ggg cgc gtg ctg ggt aaa ctc aac cag gtg ccg gat gtt atc     2208
Val Leu Gly Arg Val Leu Gly Lys Leu Asn Gln Val Pro Asp Val Ile
                725                 730                 735
gac gcg cgt cgg ttg cac ggg agt tag                                 2235
Asp Ala Arg Arg Leu His Gly Ser
            740

<210>  20
<211>  744
<212>  PRT
<213>  Escherichia coli

<400>  20
Met Val Ala Val Arg Ser Ala His Ile Asn Lys Ala Gly Glu Phe Asp
1               5                   10                  15
Pro Glu Lys Trp Ile Ala Ser Leu Gly Ile Thr Ser Gln Lys Ser Cys
            20                  25                  30
Glu Cys Leu Ala Glu Thr Trp Ala Tyr Cys Leu Gln Gln Thr Gln Gly
        35                  40                  45
His Pro Asp Ala Ser Leu Leu Leu Trp Arg Gly Val Glu Met Val Glu
    50                  55                  60
Ile Leu Ser Thr Leu Ser Met Asp Ile Asp Thr Leu Arg Ala Ala Leu
65                  70                  75                  80
Leu Phe Pro Leu Ala Asp Ala Asn Val Val Ser Glu Asp Val Leu Arg
                85                  90                  95
Glu Ser Val Gly Lys Ser Val Val Asn Leu Ile His Gly Val Arg Asp
            100                 105                 110
Met Ala Ala Ile Arg Gln Leu Lys Ala Thr His Thr Asp Ser Val Ser
        115                 120                 125
Ser Glu Gln Val Asp Asn Val Arg Arg Met Leu Leu Ala Met Val Asp
    130                 135                 140
Asp Phe Arg Cys Val Val Ile Lys Leu Ala Glu Arg Ile Ala His Leu
145                 150                 155                 160
Arg Glu Val Lys Asp Ala Pro Glu Asp Glu Arg Val Leu Ala Ala Lys
                165                 170                 175
Glu Cys Thr Asn Ile Tyr Ala Pro Leu Ala Asn Arg Leu Gly Ile Gly
            180                 185                 190
Gln Leu Lys Trp Glu Leu Glu Asp Tyr Cys Phe Arg Tyr Leu His Pro
        195                 200                 205
Thr Glu Tyr Lys Arg Ile Ala Lys Leu Leu His Glu Arg Arg Leu Asp
    210                 215                 220
Arg Glu His Tyr Ile Glu Glu Phe Val Gly His Leu Arg Ala Glu Met
225                 230                 235                 240
Lys Ala Glu Gly Val Lys Ala Glu Val Tyr Gly Arg Pro Lys His Ile

                245                 250                 255
Tyr Ser Ile Trp Arg Lys Met Gln Lys Lys Asn Leu Ala Phe Asp Glu
            260                 265                 270
Leu Phe Asp Val Arg Ala Val Arg Ile Val Ala Glu Arg Leu Gln Asp
        275                 280                 285
Cys Tyr Ala Ala Leu Gly Ile Val His Thr His Tyr Arg His Leu Pro
    290                 295                 300
Asp Glu Phe Asp Asp Tyr Val Ala Asn Pro Lys Pro Asn Gly Tyr Gln
305                 310                 315                 320
Ser Ile His Thr Val Val Leu Gly Pro Gly Gly Lys Thr Val Glu Ile
                325                 330                 335
Gln Ile Arg Thr Lys Gln Met His Glu Asp Ala Glu Leu Gly Val Ala
            340                 345                 350
Ala His Trp Lys Tyr Lys Glu Gly Ala Ala Ala Gly Gly Ala Arg Ser
        355                 360                 365
Gly His Glu Asp Arg Ile Ala Trp Leu Arg Lys Leu Ile Ala Trp Gln
    370                 375                 380
Glu Glu Met Ala Asp Ser Gly Glu Met Leu Asp Glu Val Arg Ser Gln
385                 390                 395                 400
Val Phe Asp Asp Arg Val Tyr Val Phe Thr Pro Lys Gly Asp Val Val
                405                 410                 415
Asp Leu Pro Ala Gly Ser Thr Pro Leu Asp Phe Ala Tyr His Ile His
            420                 425                 430
Ser Asp Val Gly His Arg Cys Ile Gly Ala Lys Ile Gly Gly Arg Ile
        435                 440                 445
Val Pro Phe Thr Tyr Gln Leu Gln Met Gly Asp Gln Ile Glu Ile Ile
    450                 455                 460
Thr Gln Lys Gln Pro Asn Pro Ser Arg Asp Trp Leu Asn Pro Asn Leu
465                 470                 475                 480
Gly Tyr Val Thr Thr Ser Arg Gly Arg Ser Lys Ile His Ala Trp Phe
                485                 490                 495
Arg Lys Gln Asp Arg Asp Lys Asn Ile Leu Ala Gly Arg Gln Ile Leu
            500                 505                 510
Asp Asp Glu Leu Glu His Leu Gly Ile Ser Leu Lys Glu Ala Glu Lys
        515                 520                 525
His Leu Leu Pro Arg Tyr Asn Phe Asn Asp Val Asp Glu Leu Leu Ala
    530                 535                 540
Ala Ile Gly Gly Gly Asp Ile Arg Leu Asn Gln Met Val Asn Phe Leu
545                 550                 555                 560
Gln Ser Gln Phe Asn Lys Pro Ser Ala Glu Glu Gln Asp Ala Ala Ala
                565                 570                 575
Leu Lys Gln Leu Gln Gln Lys Ser Tyr Thr Pro Gln Asn Arg Ser Lys
            580                 585                 590
Asp Asn Gly Arg Val Val Val Glu Gly Val Gly Asn Leu Met His His
        595                 600                 605

Ile Ala Arg Cys Cys Gln Pro Ile Pro Gly Asp Glu Ile Val Gly Phe
    610                 615                 620
Ile Thr Gln Gly Arg Gly Ile Ser Val His Arg Ala Asp Cys Glu Gln
625                 630                 635                 640
Leu Ala Glu Leu Arg Ser His Ala Pro Glu Arg Ile Val Asp Ala Val
                645                 650                 655
Trp Gly Glu Ser Tyr Ser Ala Gly Tyr Ser Leu Val Val Arg Val Val
            660                 665                 670
Ala Asn Asp Arg Ser Gly Leu Leu Arg Asp Ile Thr Thr Ile Leu Ala
        675                 680                 685
Asn Glu Lys Val Asn Val Leu Gly Val Ala Ser Arg Ser Asp Thr Lys
    690                 695                 700
Gln Gln Leu Ala Thr Ile Asp Met Thr Ile Glu Ile Tyr Asn Leu Gln
705                 710                 715                 720
Val Leu Gly Arg Val Leu Gly Lys Leu Asn Gln Val Pro Asp Val Ile
                725                 730                 735
Asp Ala Arg Arg Leu His Gly Ser
            740

※<210> 21
<211> 2109
<212> DNA
<213> Escherichia coli

<220>
<221> CDS
<222> (1)..(2109)

<400> 21
ttg tat ctg ttt gaa agc ctg aat caa ctg att caa acc tac ctg ccg      48
Leu Tyr Leu Phe Glu Ser Leu Asn Gln Leu Ile Gln Thr Tyr Leu Pro
1               5                   10                  15
gaa gac caa atc aag cgt ctg cgg cag gcg tat ctc gtt gca cgt gat      96
Glu Asp Gln Ile Lys Arg Leu Arg Gln Ala Tyr Leu Val Ala Arg Asp
            20                  25                  30
gct cac gag ggg caa aca cgt tca agc ggt gaa ccc tat atc acg cac      144
Ala His Glu Gly Gln Thr Arg Ser Ser Gly Glu Pro Tyr Ile Thr His
        35                  40                  45
ccg gta gcg gtt gcc tgc att ctg gcc gag atg aaa ctc gac tat gaa      192
Pro Val Ala Val Ala Cys Ile Leu Ala Glu Met Lys Leu Asp Tyr Glu
    50                  55                  60
acg ctg atg gcg gcg ctg ctg cat gac gtg att gaa gat act ccc gcc      240
Thr Leu Met Ala Ala Leu Leu His Asp Val Ile Glu Asp Thr Pro Ala
65                  70                  75                  80
acc tac cag gat atg gaa cag ctt ttt ggt aaa agc gtc gcc gag ctg      288

Thr Tyr Gln Asp Met Glu Gln Leu Phe Gly Lys Ser Val Ala Glu Leu
                85                  90                  95
gta gag ggg gtg tcg aaa ctt gat aaa ctc aag ttc cgc gat aag aaa 336
Val Glu Gly Val Ser Lys Leu Asp Lys Leu Lys Phe Arg Asp Lys Lys
            100                 105                 110
gag gcg cag gcc gaa aac ttt cgc aag atg att atg gcg atg gtg cag 384
Glu Ala Gln Ala Glu Asn Phe Arg Lys Met Ile Met Ala Met Val Gln
        115                 120                 125
gat atc cgc gtc atc ctc atc aaa ctt gcc gac cgt acc cac aac atg 432
Asp Ile Arg Val Ile Leu Ile Lys Leu Ala Asp Arg Thr His Asn Met
    130                 135                 140
cgc acg ctg ggc tca ctt cgc ccg gac aaa cgt cgc cgc atc gcc cgt 480
Arg Thr Leu Gly Ser Leu Arg Pro Asp Lys Arg Arg Arg Ile Ala Arg
145                 150                 155                 160
gaa act ctc gaa att tat agc ccg ctg gcg cac cgt tta ggt atc cac 528
Glu Thr Leu Glu Ile Tyr Ser Pro Leu Ala His Arg Leu Gly Ile His
                165                 170                 175
cac att aaa acc gaa ctc gaa gag ctg ggt ttt gag gcg ctg tat ccc 576
His Ile Lys Thr Glu Leu Glu Glu Leu Gly Phe Glu Ala Leu Tyr Pro
            180                 185                 190
aac cgt tat cgc gta atc aaa gaa gtg gtg aaa gcc gcg cgc ggc aac 624
Asn Arg Tyr Arg Val Ile Lys Glu Val Val Lys Ala Ala Arg Gly Asn
        195                 200                 205
cgt aaa gag atg atc cag aag att ctt tct gaa atc gaa ggg cgt ttg 672
Arg Lys Glu Met Ile Gln Lys Ile Leu Ser Glu Ile Glu Gly Arg Leu
    210                 215                 220
cag gaa gcg gga ata ccg tgc cgc gtc agt ggt cgc gag aag cat ctt 720
Gln Glu Ala Gly Ile Pro Cys Arg Val Ser Gly Arg Glu Lys His Leu
225                 230                 235                 240
tat tcg att tac tgc aaa atg gtg ctc aaa gag cag cgt ttt cac tcg 768
Tyr Ser Ile Tyr Cys Lys Met Val Leu Lys Glu Gln Arg Phe His Ser
                245                 250                 255
atc atg gac atc tac gct ttc cgc gtg atc gtc aat gat tct gac acc 816
Ile Met Asp Ile Tyr Ala Phe Arg Val Ile Val Asn Asp Ser Asp Thr
            260                 265                 270
tgt tat cgc gtg ctg ggc cag atg cac agc ctg tac aag ccg cgt ccg 864
Cys Tyr Arg Val Leu Gly Gln Met His Ser Leu Tyr Lys Pro Arg Pro
        275                 280                 285
ggc cgc gtg aaa gac tat atc gcc att cca aaa gcg aac ggc tat cag 912
Gly Arg Val Lys Asp Tyr Ile Ala Ile Pro Lys Ala Asn Gly Tyr Gln
    290                 295                 300
tct ttg cac acc tcg atg atc ggc ccg cac ggt gtg ccg gtt gag gtc 960
Ser Leu His Thr Ser Met Ile Gly Pro His Gly Val Pro Val Glu Val
305                 310                 315                 320
cag atc cgt acc gaa gat atg gac cag atg gcg gag atg ggt gtt gcc 1008

Gln Ile Arg Thr Glu Asp Met Asp Gln Met Ala Glu Met Gly Val Ala
                325                 330                 335
gcg cac tgg gct tat aaa gag cac ggc gaa acc agt act acc gca caa 1056
Ala His Trp Ala Tyr Lys Glu His Gly Glu Thr Ser Thr Thr Ala Gln
            340                 345                 350
atc cgc gcc cag cgc tgg atg caa agc ctg ctg gag ctg caa cag agc 1104
Ile Arg Ala Gln Arg Trp Met Gln Ser Leu Leu Glu Leu Gln Gln Ser
        355                 360                 365
gcc ggt agt tcg ttt gaa ttt atc gag agc gtt aaa tcc gat ctc ttc 1152
Ala Gly Ser Ser Phe Glu Phe Ile Glu Ser Val Lys Ser Asp Leu Phe
    370                 375                 380
ccg gat gag att tac gtt ttc aca ccg gaa ggg cgc att gtc gag ctg 1200
Pro Asp Glu Ile Tyr Val Phe Thr Pro Glu Gly Arg Ile Val Glu Leu
385                 390                 395                 400
cct gcc ggt gca acg ccc gtc gac ttc gct tat gca gtg cat acc gat 1248
Pro Ala Gly Ala Thr Pro Val Asp Phe Ala Tyr Ala Val His Thr Asp
                405                 410                 415
atc ggt cat gcc tgc gtg ggc gca cgc gtt gac cgc cag cct tac ccg 1296
Ile Gly His Ala Cys Val Gly Ala Arg Val Asp Arg Gln Pro Tyr Pro
            420                 425                 430
ctg tcg cag ccg ctt acc agc ggt caa acc gtt gaa atc att acc gct 1344
Leu Ser Gln Pro Leu Thr Ser Gly Gln Thr Val Glu Ile Ile Thr Ala
        435                 440                 445
ccg ggc gct cgc ccg aat gcc gct tgg ctg aac ttt gtc gtt agc tcg 1392
Pro Gly Ala Arg Pro Asn Ala Ala Trp Leu Asn Phe Val Val Ser Ser
    450                 455                 460
aaa gcg cgc gcc aaa att cgt cag ttg ctg aaa aac ctc aag cgt gat 1440
Lys Ala Arg Ala Lys Ile Arg Gln Leu Leu Lys Asn Leu Lys Arg Asp
465                 470                 475                 480
gat tct gta agc ctg ggc cgt cgt ctg ctc aac cat gct ttg ggt ggt 1488
Asp Ser Val Ser Leu Gly Arg Arg Leu Leu Asn His Ala Leu Gly Gly
                485                 490                 495
agc cgt aag ctg aat gaa atc ccg cag gaa aat att cag cgc gag ctg 1536
Ser Arg Lys Leu Asn Glu Ile Pro Gln Glu Asn Ile Gln Arg Glu Leu
            500                 505                 510
gat cgc atg aag ctg gca acg ctt gac gat ctg ctg gca gaa atc gga 1584
Asp Arg Met Lys Leu Ala Thr Leu Asp Asp Leu Leu Ala Glu Ile Gly
        515                 520                 525
ctt ggt aac gca atg agc gtg gtg gtc gcg aaa aat ctg caa cat ggg 1632
Leu Gly Asn Ala Met Ser Val Val Val Ala Lys Asn Leu Gln His Gly
    530                 535                 540
gac gcc tcc att cca ccg gca acc caa agc cac gga cat ctg ccc att 1680
Asp Ala Ser Ile Pro Pro Ala Thr Gln Ser His Gly His Leu Pro Ile
545                 550                 555                 560
aaa ggt gcc gat ggc gtg ctg atc acc ttt gcg aaa tgc tgc cgc cct 1728

Lys Gly Ala Asp Gly Val Leu Ile Thr Phe Ala Lys Cys Cys Arg Pro
                565                 570                 575
att cct ggc gac ccg att atc gcc cac gtc agc ccc ggt aaa ggt ctg 1776
Ile Pro Gly Asp Pro Ile Ile Ala His Val Ser Pro Gly Lys Gly Leu
            580                 585                 590
gtg atc cac cat gaa tcc tgc cgt aat atc cgt ggc tac cag aaa gag 1824
Val Ile His His Glu Ser Cys Arg Asn Ile Arg Gly Tyr Gln Lys Glu
        595                 600                 605
cca gag aag ttt atg gct gtg gaa tgg gat aaa gag acg gcg cag gag 1872
Pro Glu Lys Phe Met Ala Val Glu Trp Asp Lys Glu Thr Ala Gln Glu
    610                 615                 620
ttc atc acc gaa atc aag gtg gag atg ttc aat cat cag ggt gcg ctg 1920
Phe Ile Thr Glu Ile Lys Val Glu Met Phe Asn His Gln Gly Ala Leu
625                 630                 635                 640
gca aac ctg acg gcg gca att aac acc acg act tcg aat att caa agt 1968
Ala Asn Leu Thr Ala Ala Ile Asn Thr Thr Thr Ser Asn Ile Gln Ser
                645                 650                 655
ttg aat acg gaa gag aaa gat ggt cgc gtc tac agc gcc ttt att cgt 2016
Leu Asn Thr Glu Glu Lys Asp Gly Arg Val Tyr Ser Ala Phe Ile Arg
            660                 665                 670
ctg acc gct cgt gac cgt gtg cat ctg gcg aat atc atg cgc aaa atc 2064
Leu Thr Ala Arg Asp Arg Val His Leu Ala Asn Ile Met Arg Lys Ile
        675                 680                 685
cgc gtg atg cca gac gtg att aaa gtc acc cga aac cga aat taa 2109
Arg Val Met Pro Asp Val Ile Lys Val Thr Arg Asn Arg Asn
    690                 695                 700

<210> 22
<211> 702
<212> PRT
<213> Escherichia coli

<400> 22
Leu Tyr Leu Phe Glu Ser Leu Asn Gln Leu Ile Gln Thr Tyr Leu Pro
1               5                   10                  15
Glu Asp Gln Ile Lys Arg Leu Arg Gln Ala Tyr Leu Val Ala Arg Asp
            20                  25                  30
Ala His Glu Gly Gln Thr Arg Ser Ser Gly Glu Pro Tyr Ile Thr His
        35                  40                  45
Pro Val Ala Val Ala Cys Ile Leu Ala Glu Met Lys Leu Asp Tyr Glu
    50                  55                  60
Thr Leu Met Ala Ala Leu Leu His Asp Val Ile Glu Asp Thr Pro Ala
65                  70                  75                  80
Thr Tyr Gln Asp Met Glu Gln Leu Phe Gly Lys Ser Val Ala Glu Leu
                85                  90                  95

Val Glu Gly Val Ser Lys Leu Asp Lys Leu Lys Phe Arg Asp Lys Lys
            100                 105                 110
Glu Ala Gln Ala Glu Asn Phe Arg Lys Met Ile Met Ala Met Val Gln
        115                 120                 125
Asp Ile Arg Val Ile Leu Ile Lys Leu Ala Asp Arg Thr His Asn Met
    130                 135                 140
Arg Thr Leu Gly Ser Leu Arg Pro Asp Lys Arg Arg Arg Ile Ala Arg
145                 150                 155                 160
Glu Thr Leu Glu Ile Tyr Ser Pro Leu Ala His Arg Leu Gly Ile His
                165                 170                 175
His Ile Lys Thr Glu Leu Glu Glu Leu Gly Phe Glu Ala Leu Tyr Pro
            180                 185                 190
Asn Arg Tyr Arg Val Ile Lys Glu Val Val Lys Ala Ala Arg Gly Asn
        195                 200                 205
Arg Lys Glu Met Ile Gln Lys Ile Leu Ser Glu Ile Glu Gly Arg Leu
    210                 215                 220
Gln Glu Ala Gly Ile Pro Cys Arg Val Ser Gly Arg Glu Lys His Leu
225                 230                 235                 240
Tyr Ser Ile Tyr Cys Lys Met Val Leu Lys Glu Gln Arg Phe His Ser
                245                 250                 255
Ile Met Asp Ile Tyr Ala Phe Arg Val Ile Val Asn Asp Ser Asp Thr
            260                 265                 270
Cys Tyr Arg Val Leu Gly Gln Met His Ser Leu Tyr Lys Pro Arg Pro
        275                 280                 285
Gly Arg Val Lys Asp Tyr Ile Ala Ile Pro Lys Ala Asn Gly Tyr Gln
    290                 295                 300
Ser Leu His Thr Ser Met Ile Gly Pro His Gly Val Pro Val Glu Val
305                 310                 315                 320
Gln Ile Arg Thr Glu Asp Met Asp Gln Met Ala Glu Met Gly Val Ala
                325                 330                 335
Ala His Trp Ala Tyr Lys Glu His Gly Glu Thr Ser Thr Thr Ala Gln
            340                 345                 350
Ile Arg Ala Gln Arg Trp Met Gln Ser Leu Leu Glu Leu Gln Gln Ser
        355                 360                 365
Ala Gly Ser Ser Phe Glu Phe Ile Glu Ser Val Lys Ser Asp Leu Phe
    370                 375                 380
Pro Asp Glu Ile Tyr Val Phe Thr Pro Glu Gly Arg Ile Val Glu Leu
385                 390                 395                 400
Pro Ala Gly Ala Thr Pro Val Asp Phe Ala Tyr Ala Val His Thr Asp
                405                 410                 415
Ile Gly His Ala Cys Val Gly Ala Arg Val Asp Arg Gln Pro Tyr Pro
            420                 425                 430
Leu Ser Gln Pro Leu Thr Ser Gly Gln Thr Val Glu Ile Ile Thr Ala
        435                 440                 445
Pro Gly Ala Arg Pro Asn Ala Ala Trp Leu Asn Phe Val Val Ser Ser

450                 455                 460
Lys Ala Arg Ala Lys Ile Arg Gln Leu Leu Lys Asn Leu Lys Arg Asp
465                 470                 475                 480
Asp Ser Val Ser Leu Gly Arg Arg Leu Leu Asn His Ala Leu Gly Gly
                485                 490                 495
Ser Arg Lys Leu Asn Glu Ile Pro Gln Glu Asn Ile Gln Arg Glu Leu
            500                 505                 510
Asp Arg Met Lys Leu Ala Thr Leu Asp Asp Leu Leu Ala Glu Ile Gly
        515                 520                 525
Leu Gly Asn Ala Met Ser Val Val Val Ala Lys Asn Leu Gln His Gly
    530                 535                 540
Asp Ala Ser Ile Pro Pro Ala Thr Gln Ser His Gly His Leu Pro Ile
545                 550                 555                 560
Lys Gly Ala Asp Gly Val Leu Ile Thr Phe Ala Lys Cys Cys Arg Pro
                565                 570                 575
Ile Pro Gly Asp Pro Ile Ile Ala His Val Ser Pro Gly Lys Gly Leu
            580                 585                 590
Val Ile His His Glu Ser Cys Arg Asn Ile Arg Gly Tyr Gln Lys Glu
        595                 600                 605
Pro Glu Lys Phe Met Ala Val Glu Trp Asp Lys Glu Thr Ala Gln Glu
    610                 615                 620
Phe Ile Thr Glu Ile Lys Val Glu Met Phe Asn His Gln Gly Ala Leu
625                 630                 635                 640
Ala Asn Leu Thr Ala Ala Ile Asn Thr Thr Thr Ser Asn Ile Gln Ser
                645                 650                 655
Leu Asn Thr Glu Glu Lys Asp Gly Arg Val Tyr Ser Ala Phe Ile Arg
            660                 665                 670
Leu Thr Ala Arg Asp Arg Val His Leu Ala Asn Ile Met Arg Lys Ile
        675                 680                 685
Arg Val Met Pro Asp Val Ile Lys Val Thr Arg Asn Arg Asn
    690                 695                 700

## Description

### BACKGROUND OF THE INVENTION

### Field of the Invention

The present invention relates to the fermentation industry. More specifically, the present invention relates to a bacterium having an ability to produce an L-amino acid and a method for producing an L-amino acid utilizing such a bacterium.

### Description of the Related Art

To date, it has been widely clarified that ppGpp (guanosine-3'-diphosphate-5'-diphosphate) and pppGpp (guanosine-3'-triphosphate-5'-diphosphate) play important roles as signal materials in microbial cells. It is known that ppGpp is an essential substance for inducing adaptation apparatuses of microorganisms to survive under conditions under which intracellular amino acids and sugars necessary for their proliferation are exhausted.

It has been reported that ppGpp is produced by the RelA protein that is a gene product of *relA* gene and by the SpoT protein that is a gene product of *spoT* gene in *Escherichia coli.* In addition, nucleotide sequences and amino acid sequences of these gene and proteins have also been reported (GenBank accession J04039, Metzger,S. et al., J. Biol. Chem., 1988, 263 (30), 15699-15704, GenBank accession AE000442 U00096).

The RelA protein is present in a bacterial cell in a form of binding to a ribosome, and it receives a signal of amino acid depletion as a result of binding of a non-aminoacylated tRNA to a ribosome and produces pppGpp from GTP and GDP. On the other hand, it is known that the SpoT protein is an enzyme that catalyzes three kinds of reactions, i.e., the reaction from pppGpp to ppGpp, reaction from GTP to ppGpp and reaction from ppGpp to GTP. Hereinafter, both ppGpp and pppGpp are collectively referred to as "ppGpp", because it is thought that physiological functions of those in a cell are identical.

It is known that when ppGpp is accumulated in cells by the RelA protein due to sudden depletion of amino acids in a medium and cells, the cells generally exhibit a series of responses including termination of ribosome synthesis, degradation of ribosomal proteins, promotion of expression of genes in various amino acid biosynthetic pathways and so forth. These are generally referred to as stringent responses, and it is widely known that they are responses necessary for the cells to survive under starvation conditions by supplying depleted amino acids (Cashel, M., Gentry, D.R., Hernadez, V.J., and Vinella, D., The stringent response, In: Neidhardt, FC. et al. (ed) *Escherichia coli* and *Salmonella;* Cellar and Molecular Biology, 2^{nd} edition, 1458-1496 (ASM Press, Washington D.C., 1996).

Examples of analysis conducted to date with paying attentions to ppGpp concerning material production include improving production of a protein utilizing a recombinant *Escherichia coli* by eliminating an ability to produce ppGpp (Dedhia, N. et al., Biotechnol. Bioeng., 1997, Vol. 53,379-386), improving productivity of antibiotics by modifying ppGpp-binding sites of ribosomal proteins and RNA polymerase in Actinomyces (Hu, H. and Ochi, K., Appl. Environ. Microbiol., 2001, Vol. 67, 1885-18921, Hu, H., Zhang, Q., and Ochi, K., J. Bacteriol., 2002, Vol. 184, 3984-3991) and so forth.

However, to date, no research has been reported concerning the relationship between amino acid biosynthetic systems, which are widely known to be directly acted by ppGpp, and the ability to produce ppGpp.

### SUMMARY OF THE INVENTION

An object of the present invention is to improve an ability to produce an L-amino acid of a bacterium and a bacterium having an improved ability to produce an L-amino acid.

It is an object of the present invention to provide a method for producing an L-amino acid comprising culturing a bacterium having an ability to produce an L-amino acid in a medium to allow accumulation of the L-amino acid in a culture, and collecting the L-amino acid from the culture, wherein the bacterium is modified so that an activity to synthesize ppGpp is increased.

It is a further object of the present invention to provide the method as described above, wherein the bacterium is modified so that an activity of an enzyme synthesizing ppGpp is increased.

It is a further object of the present invention to provide the method as described above, wherein the enzyme is a RelA protein or a catalytic domain of the RelA protein.

It is a further object of the present invention to provide the method as described above, wherein the activity of the RelA protein is increased by increasing copy number of a *relA* gene or a partial region of the *relA* gene encoding a catalytic domain of the RelA protein, or modifying an expression regulatory sequence of the *relA* gene so that intracellular expression of the *relA* gene or a partial region of the *relA* gene encoding a catalytic domain of the RelA protein of the bacterium is enhanced.

It is a further object of the present invention to provide the method as described above, wherein the RelA protein is a protein defined in the following (A) or (B):
(A) a protein which has the amino acid sequence of SEQ ID NO: 20.
(B) a protein which has the amino acid sequence of SEQ ID NO: 20 including substitution, deletion, insertion or addition of one or several amino acid residues,
and wherein the protein has an activity to synthesize ppGpp.

It is a further object of the present invention to provide the method as described above, wherein the RelA protein is encoded by a DNA defined in the following (a) or (b):
(a) a DNA which has the nucleotide sequence of SEQ ID NO: 19,
(b) a DNA which is hybridizable with the nucleotide sequence of SEQ ID NO: 19 under a stringent condition,
and wherein said DNA encodes a protein which has an acitivity to syntehsize ppGpp.

It is a further object of the present invention to provide the method as described above, wherein the L-amino acid is selected from the group consisting of L-glutamic acid, L-threonine, L-isoleucine, L-lysine, L-histidine, L-valine, L-arginine, L-leucine, L-phenylalanine and L-triptophan.

It is a further object of the present invention to provide the method as described above, wherein the L-amino acid is selected from the group consisting of L-glutamic acid, L-threonine, L-isoleucine or L-lysine.

It is a further object of the present invention to provide the method as described above, wherein the bacterium is a bacterium belonging to the genus *Escherichia.*

It is a still further object of the present invention to provide a bacterium which has an ability to produce an L-amino acid and modified so that an activity of intracellular RelA protein is increased.

According to the present invention, L-amino acid production of bacteria can be improved.

### BRIEF DESCRIPTION OF THE DRAWINGS

Fig. 1 shows construction of plasmids pM14 and pM15.
Fig. 2 shows construction of plasmid pMD4041-cat-2Tfd.
Fig. 3 shows construction of plasmids pSTVrelA and pSTVrelA*.

### DESCRIPTION OF THE PREFERRED EMBODIMENTS

The inventors of the present invention assiduously studied in order to achieve the aforementioned objects. As a result, it was found that an ability to produce an L-amino acid can be enhanced by increasing an ability to produce ppGpp, in particular, by increasing an activity of the RelA protein to synthesize ppGpp. Thus, they accomplished the present invention.

Hereafter, the present invention will be explained in detail.

### Bacterium of the present invention

The bacterium of the present invention is a bacterium which has an ability to produce an L-amino acid and which is modified so that an ability to synthesize ppGpp in the cell is increased.

The bacterium of the present invention is not particularly limited so long as an ability to produce an L-amino acid of the bacterium can be increased by increasing the ability to synthesize ppGpp. Examples of the bacterium include, but are not limited to, bacteria belonging to the genus *Escherichia* such as *Escherichia coli,* coryneform bacteria such as *Brevibacterium lactofermentum,* bacteria belonging to the genus *Serratia* such as *Serratia marcescens,* bacteria belonging to the genus *Bacillus* such as *Bacillus subtilis* and so forth.

The term "an ability to produce an L-amino acid" used in the present invention means an ability to cause accumulation of the L-amino acid in a medium when the bacterium of the present invention is cultured in the medium. This ability to produce an L-amino acid may be an inherent property of a wild-type strain of a bacterium or a property imparted or enhanced by breeding.

Examples of the L-amino acid include L-glutamic acid, L-threonine, L-isoleucine, L-lysine, L-histidine, L-valine, L-arginine, L-leucine, L-phenylalanine, L-triptophan and so forth. Among these, L-glutamic acid, L-threonine, L-isoleucine and L-lysine are preferred.

Specific examples of the bacterium having an ability to produce an L-amino acid include, but are not limited to, the followings: for L-glutamic acid as the target L-amino acid, the *Escherichia coli* MG1655ΔsucA (see the examples section), *Escherichia coli* AJ12624 (FERM BP-3853, see French Patent Laid-open Publication No. 2,680,178) and L-valine resistant strains of *Escherichia coli* such as *Escherichia coli* B11, *Escherichia coli* K-12 (ATCC10798), *Escherichia coli* B (ATCC11303) and *Escherichia coli* W (ATCC9637), *Brevibacterium lactofermentum* AJ12475 (FERM BP-2922, see U.S. Patent No. 5,272,067) and so forth;
for L-threonine, *Escherichia coli* VKPM B-3996 (see U.S. Patent No. 5,175,107), *Escherichia coli* MG442 strain (VKPM B-1628, see Gusyatiner et al., Genetika (in Russian), 14, pp.947-956, 1978, U.S. Patent No. 4,278,765), *Corynebacterium acetoacidophilum* AJ12318 (FERM BP-1172, see U.S. Patent No. 5,188,949) and so forth,
for L-isoleucine, *Escherichia coli* KX141 (VKPM B-4781, see European Patent Application Laid-open No. 519,113), *Brevibacterium flavum* AJ12149 (PERM BP-759, see U.S. Patent No. 4,656,135) and so forth,
for L-lysine, *Escherichia coli* AJ11442 (NRRL B-12185, FERM BP-1543, see U.S. Patent No. 4,346,170), *Escherichia coli* WC196 strain (FERM BP-5252, WO96/17930), *Brevibacterium lactofermentum* AJ3990 (ATCC31269, see U.S. Patent No. 4,066,501) and so forth,
for L-phenylalanine, *Escherichia coli* AJ 12604 (PERM BP-3579, see European Patent Application Laid-open No. 488,424), *Brevibacterium lactofermentum* AJ12637 (FERM BP-4160, see French Patent Application Laid-open No. 2,686,898) and so forth,
for L-leucine, strains having β-2-thienylalanine resistance, strains having resistance to β-2-thienylalanine and β-hydroxyleucine (see Japanese Patent Publication (Kokoku) No. 62-34397 for all of the above), strains having resistance to 4-azaleucine resistance or 5,5,5-trifluoroleucine (Japanese Patent Laid-open (Kokai) No. 8-70879), *Escherichia coli* AJ11478 (FERM P-5274, see Japanese Patent Publication No. 62-34397), *Brevibacterium lactofermentum* AJ3718 (FERM P-2516, see U.S. Patent No. 3,970,519) and so forth,
for L-valine, *Escherichia coli* VL1970 (VKPM B-4411, (see European Patent Application Laid-open No. 519,113), *Brevibacterium lactofermentum* AJ12341 (FERM BP-1763, see U.S. Patent No. 5,188,948) and so forth, and
for L-homoserine, the NZ10 strain, which is a Leu⁺ revertant of the *Escherichia coli* C600 strain (see Appleyard R.K., Genetics, 39, pp.440-452, 1954).

Among the above strains, the *Escherichia coli* MG1655ΔsucA is a strain obtained by disrupting a gene *(sucA)* encoding the E1 subunit of aKGDH (a-ketoglutarate dehydrogenase) of the MG1655 strain (available from E. coli Genetic Stock Center (Yale University, Dept. Biology, Osborn Memorial Labs., 06511-7444 New Haven, Connecticut, U.S.A., P.O. Box 6666) (see examples section). The nucleotide sequence and the amino acid sequence encoded thereby are known (see, for example, GenBank accession X00661). Furthermore, disruption of choromosomal *sucA* gene of *Escherichia coli* has been known (see EP 0 670 370 B1).

Furthermore, the *Escherichia coli* B-3996 strain is deficient in the *thrC* gene, utilizes sucrose and has a leaky mutation in the *ilvA* gene. This strain has a mutation in the *rht* gene involved in the high resistance to threonine and homoserine (French Patent Application Laid-open No. 2804971). The B-3996 strain harbors the plasmid pVIC40 obtained by inserting a *thrA***BC* operon containing a mutant *thrA* gene encoding aspartokinase-homoserine dehydrogenase I for which feedback inhibition by threonine is substantially desensitized into a vector derived from RSF1010. The B-3996 strain was deposited at the Russian National Collection of Industrial Microorganisms (VKPM) (Address: Dorozhny proezd. 1, Moscow 113545, Russian Federation) on April 7, 1987 and received an accession number of B-3996.

B-3996/pMWD5 (Japanese Patent Laid-open No. 08-047397, U.S. Patent No. 5,998,178) is a strain obtained by introducing a plasmid pMWD5 containing the *ilvGMEDA* operon of which region required for attenuation is removed (Japanese Patent Laid-open No. 08-047397, WO96/26289) into the B-3996 strain.

The bacterium of the present invention can be obtained by modifying a bacterium having an ability to produce an L-amino acid such as those mentioned above so that the activity to synthesize ppGpp of the bacterium is increased. The bacterium of the present invention can also be obtained by imparting an ability to produce an L-amino acid to a bacterium modified so that the activity to synthesize ppGpp of the bacterium is increased, or enhancing an ability to produce an L-amino acid of such a bacterium.

The expression "modified so that the activity to synthesize ppGpp is increased" means that the activity to synthesize ppGpp per cell is increased compared with that of a non-modified strain, e.g., a wild-type strain. Examples of such a wild-type strain include, but are not limited to, *Escherichia coli* MG1655 as for *Escherichia coli.*

The activity to synthesize ppGpp of a bacterium can be increased by modifying the bacterium so that an activity of an enzyme to synthesize ppGpp is increased. Examples of the ppGpp synthesis enzyme include RelA protein and SpoT protein. Among these, the RelA protein is preferred. Furthermore, a bacterium may be modified so that the activities of both of RelA protein and SpoT protein is increased.

The activities of the aforementioned bacterial proteins can be increased by, for example, enhancing the expression of the gene encoding RelA (*relA*) or gene encoding SpoT *(spoT).* Enhancement of expression levels of these genes can be achieved by increasing copy number of *relA* or spoT. For example, a gene fragment containing *relA* or *spoT* can be ligated to a vector that functions in a bacterium, preferably a multi-copy type vector to prepare recombinant DNA and used to transform the bacterium.

Origins of the *relA* gene and *spoT* gene are not particularly limited so long as the genes function in the host bacterium to which these genes are introduced. However, a gene derived from the same species as the host or an analogous species is preferred.

The nucleotide sequences of *relA* and *spoT* of *Escherichia coli* are known *(relA :* GenBank accession AE000362, nucleotide numbers 1667 to 3901, *spoT:* GenBank accession AE000442 U00096, nucleotide numbers 3791 to 5899). The genes can be obtained by PCR (polymerase chain reaction, see White, T.J. et al., Trends Genet. 5, 185 (1989)) using primers prepared on the basis of the known nucleotide sequences and a chromosomal DNA of a bacterium belonging to the genus *Escherichia. relA* and *spoT* homologues of other microorganisms can also be obtained in a similar manner.

The nucleotide sequence of the *relA* gene and the amino acid sequence of the RelA protein of *Escherichia coli* are shown in SEQ ID NOS: 19 and 20. The nucleotide sequence of the *spoT* gene and the amino acid sequence of the SpoT protein of *Escherichia coli* are shown in SEQ ID NOS: 21 and 22.

*relA* and *spoT* used for the present invention may encode RelA or SpoT including substitution, deletion, insertion, addition or inversion of one or several amino acid residues so long as the activity to synthesize ppGpp of the encoded RelA protein and SpoT protein is not substantially degraded. Although the number of "several" amino acid residues referred to herein differs depending on positions in the three-dimensional structure of the proteins or types of amino acid residues, it may be specifically 2 to 500, preferably 2 to 100, more preferably 2 to 20.

Therefore, changes to RelA or SpoT such as those described above are typically conservative changes so as to maintain the activity of RelA or SpoT. Substitution changes include those in which at least one residue in the amino acid sequence has been removed and a different residue inserted in its place. Examples of amino acids which may be substituted for an original amino acid in a RelA or SpoT protein and which are regarded as conservative subsitutions include: Ala substituted with ser or thr; arg substituted with gln, his, or lys; asn substituted with glu, gln, lys, his, asp; asp substituted with asn, glu, or gln; cys substituted with ser or ala; gln substituted with asn, glu, lys, his, asp, or arg; glu substituted with asn, gln, lys, or asp; gly substituted with pro; his substituted with asn, lys, gln, arg, tyr; ile substituted with leu, met, val, phe; leu substituted with ile, met, val, phe; lys substituted with asn, glu, gln, his, arg; met substituted with ile, leu, val, phe; phe substituted with trp, tyr, met, ile, or leu; ser substituted with thr, ala; thr substituted with ser or ala; trp substituted with phe, tyr; tyr substituted with his, phe, or trp; and val substituted with met, ile, leu.

The RelA protein consists of a catalytic domain and a ribosome-binding domain. The ribosome-binding domain of the RelA protein may be deleted in the present invention. In the amino acid sequence of the RelA protein shown in SEQ ID NO: 20, the catalytic domain corresponds to the amino acid numbers 1 to 464. Such a RelA protein consisting only of the catalytic domain also falls within the scope of the RelA protein referred to in the present invention. In the present specification, a gene encoding a RelA protein consisting only of the catalytic domain may be also described as "*relA**".

A DNA encoding a protein substantially identical to RelA or SpoT can be obtained by modifying the nucleotide sequence of the *relA* or *spoT.* For example, site-directed mutagenesis can be employed so that substitution, deletion, insertion, addition or inversion of amino acid residues at a specific site of RelA or SpoT. Furthermore, a DNA modified as described above may also be obtained by a conventionally known mutagenesis treatments. The mutagenesis treatment includes a method of treating a DNA before the mutagenesis treatment *in vitro* with hydroxylamine or the like, and a method of treating a microorganism such as an *Escherichia* bacterium harboring a DNA before the mutagenesis treatment by ultraviolet irradiation or with a mutagenizing agent used for a usual mutagenesis treatment such as N-methyl-N'-nitro-N-nitrosoguanidine (NTG) and nitrous acid.

A DNA having such a mutation as described above can be expressed in an appropriate cell, and activity of the expression product can be investigated to obtain a DNA encoding a protein substantially identical to RelA or SpoT. A DNA encoding RelA or SpoT having a mutation can also be obtained by isolating a DNA that is hybridizable with a probe having a nucleotide sequence comprising, for example, the nucleotide sequence of SEQ ID NO: 19 or 21 or a part thereof, under stringent conditions, and encodes a protein having the activity to synthesize ppGpp from a cell harboring the DNA encoding RelA or SpoT having a mutation. The "stringent conditions" referred to herein include conditions under which so-called specific hybrid is formed, and non-specific hybrid is not formed. It is difficult to clearly express this condition using any numerical value. However, for example, the stringent conditions include conditions under which DNAs having high homology, for example, DNAs having homology of not less than 50%, hybridize with each other, but DNAs having homology lower than the above do not hybridize with each other. Alternatively, the stringent conditions are exemplified by a condition whereby DNAs hybridize with each other at a salt concentration corresponding to an ordinary condition of washing in Southern hybridization, i.e., 1 x SSC, 0.1% SDS, preferably 0.1 x SSC, 0.1% SDS, at 60°C.

A partial sequence of the nucleotide sequence of SEQ ID NO: 19 or 21 can also be used as a probe. Probes can be generated by PCR using oligonucleotides produced on the basis of the nucleotide sequence of SEQ ID NO: 19 or 20 as primers and a DNA fragment containing the nucleotide sequence of SEQ ID NO: 19 or 21 as a template. When a DNA fragment in a length of about 300 bp is used as the probe, the conditions of washing for the hybridization can be, for example, 50°C, 2 x SSC and 0.1% SDS.

Specific examples of the DNA encoding a protein substantially identical to RelA include DNA encoding a protein that has homology of preferably 70% or more, more preferably 80% or more, still more preferably 90% or more, particularly preferably 95% or more, with respect to the amino acid sequence shown in SEQ ID NO: 20 and has an activity similar to that of RelA. When the RelA protein consists only of the catalytic domain, it is preferable that the catalytic domain should have a homology of the aforementioned degree. Specific examples of the DNA encoding a protein substantially identical to SpoT include DNA encoding a protein that has homology of preferably 70% or more, more preferably 80% or more, still more preferably 90% or more, particularly preferably 95% or more, with respect to the amino acid sequence shown in SEQ ID NO: 22 and has an activity similar to that of SpoT.

A chromosomal DNA as a material for isolating RelA or SpoT can be prepared from a bacterium, which is a DNA donor, by the method of, for example, Saito and Miura (see H. Saito and K. Miura, Biochem. Biophys. Acta, 72, 619 (1963), Text for Bioengineering Experiments, Edited by the Society for Bioscience and Bioengineering, Japan, pp.97-98, Baifukan, 1992) or the like.

Examples of primer for *relA* amplification include relA5 and relA6 described in Table 1, and examples of primer for *relA** amplification include relA5 and relA7. Furthermore, examples of primer for *spoT* amplification include spoT1 and spoT4.

If a DNA fragment containing *relA* or *spoT* amplified by the PCR is ligated to a vector DNA autonomously replicable in a cell of *Escherichia coli* or the like to prepare a recombinant DNA, subsequent procedures become easier. Examples of the vector autonomously replicable in a cell of *Escherichia coli* include pUC19, pUC18, pHSG299, pHSG399, pHSG398, RSF1010, pBR322, pACYC184, pMW219, pSTV29 and so forth.

In order to prepare a recombinant DNA by ligating *relA* or *spoT* and a vector, the vector can be digested with a restriction enzyme corresponding to the terminus of the genes and ligated using a ligase such as T4 DNA ligase.

In order to introduce the recombinant DNA prepared as described above into a coryneform bacterium, any known transformation methods that have hitherto been reported can be employed. For instance, the methods include a method of treating recipient cells with calcium chloride so as to increase the permeability of DNA, which has been reported for *Escherichia coli* K-12 (Mandel, M. and Higa, A., J. Mol. Biol., 53, 159 (1970)), and a method of preparing competent cells from cells which are at the growth phase followed by introducing the DNA thereinto, which has been reported for *Bacillus subtilis* (Duncan, C.H., Wilson, G.A. and Young, F.E., Gene, 1, 153 (1977)). Furthermore, the methods of transformation include a method of making DNA-recipient cells into protoplasts or spheroplasts, which can easily take up a recombinant DNA, followed by introducing the recombinant DNA into the cells, which is known to be applicable to *Bacillus subtilis,* actinomycetes and yeasts (Chang, S. and Choen, S.N., Molec. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Sci., USA, 75, 1929 (1978)). The transformation can also be performed by the electric pulse method (Japanese Patent Laid-open No. 2-207791).

Increasing copy number of a gene can also be accomplished by introducing multiple copies of the gene into a chromosomal DNA of a bacterium. Multiple copies of the gene may be introduced into a chromosomal DNA of a bacterium by homologous recombination. This can be performed by targeting a sequence present on chromosomal DNA in multiple copy number. A repetitive DNA or an inverted repeats present at the end of a transposable element can be used as the sequences present on chromosomal DNA in multiple copy number. Alternatively, as disclosed in Japanese Patent Laid-open No. 2-109985, multiple copies of the desired gene can be introduced into chromosomal DNA by incorporating them into a transposon and transferring it.

Besides the above gene amplification methods, RelA or SpoT activity can be enhanced by replacing an expression control sequence, such as promoters of *relA* or *spoT* on a chromosomal DNA or plasmid, with stronger ones. Examples of strong promoters include *lac* promoter, *trp* promoter, *trc* promoter and so forth. Furthermore, as disclosed in International Patent Publication WO00/18935, by introducing a substitution of several nucleotides into the promoter region of the gene, the promoter can be modified so to become stronger. Substitution or modification of these promoters enhances expression of the *relA* or *spoT* gene, and thus activities of RelA and/or SpoT are enhanced. Modification of expression control sequence may be combined with the increase in copy number of the genes.

Substitution of an expression control sequence can be performed, for example, in the same manner as the gene substitution using a temperature sensitive plasmid described later. Examples of the temperature sensitive plasmid of a bacterium belonging to the genus *Escherichia* include pMAN031 (Yasueda, H. et al, Appl. Microbiol. Biotechnol., 36,211 (1991)), pMAN997 (WO 99/03988), pEL3 (K.A. Armstrong et. al., J. Mol. Biol. (1984) 175, 331-347) and so forth. pMAN997 is obtained by exchanging the VspI-*HindIII* fragments of pMAN031 (J. Bacteriol., 162, 1196 (1985)) and pUC19 (Takara Shuzo). These plasmids can autonomously replicate at least at a temperature of 30°C, but cannot autonomously replicate at a temperature of 42°C, in *Escherichia coli.*

### <2> Method for producing L-amino acid according to the present invention

An L-amino acid can be produced by culturing the bacterium of the present invention obtained as described above in a medium to produce and accumulate an L-amino acid in culture and collecting the L-amino acid from the culture.

The medium used in the present invention may be a conventionally used well-known medium selected depending on type of the bacterium to be utilized or the target L-amino acid. That is, the medium may be a typical medium containing a carbon source, nitrogen source, inorganic ions, as well as other organic components, if necessary. Any special medium is not required for practicing the present invention.

Sugars such as glucose, lactose, galactose, fructose or starch hydrolysate, alcohols such as glycerol or sorbitol, organic acids such as fumaric acid, citric acid or succinic acid and so forth can be used as the carbon source.

Inorganic ammonium salts such as ammonium sulfate, ammonium chloride or ammonium phosphate, organic nitrogen such as soybean hydrolysate, ammonia gas, aqueous ammonia and so forth ca be used as the nitrogen source.

It is desirable to allow required substances such as vitamin B₁, L-homoserine and L-tyrosine or yeast extract to be contained in appropriate amounts as organic trace nutrients. Other than the above, potassium phosphate, magnesium sulfate, iron ion, manganese ion and so forth are added in small amounts, if necessary.

The culture can be performed under conventionally used well-known conditions selected according to a strain to be utilized. For example, the culture is preferably performed under aerobic conditions for between 16 and 120 hours. The culture temperature is preferably controlled to be between 25°C and 45°C, and pH is preferably controlled at between 5 and 8 during the culture. Inorganic or organic, acidic or alkaline substances as well as ammonia gas or the like can be used for pH adjustment.

For collection of the L-amino acid from the medium after completion of the culture, any special methods are not required for the present invention. That is, collection of the target L-amino acid can be performed using a combination of conventionally well-known ion exchange techniques, precipitation techniques and other techniques depending on the type of the target L-amino acid.

### Examples

Hereinafter, the present invention will be explained more specifically with reference to the following non-limiting examples.

The amino acids referred to in the following examples are L-amino acids. The primers for PCR used in the following examples are shown in Table 1.

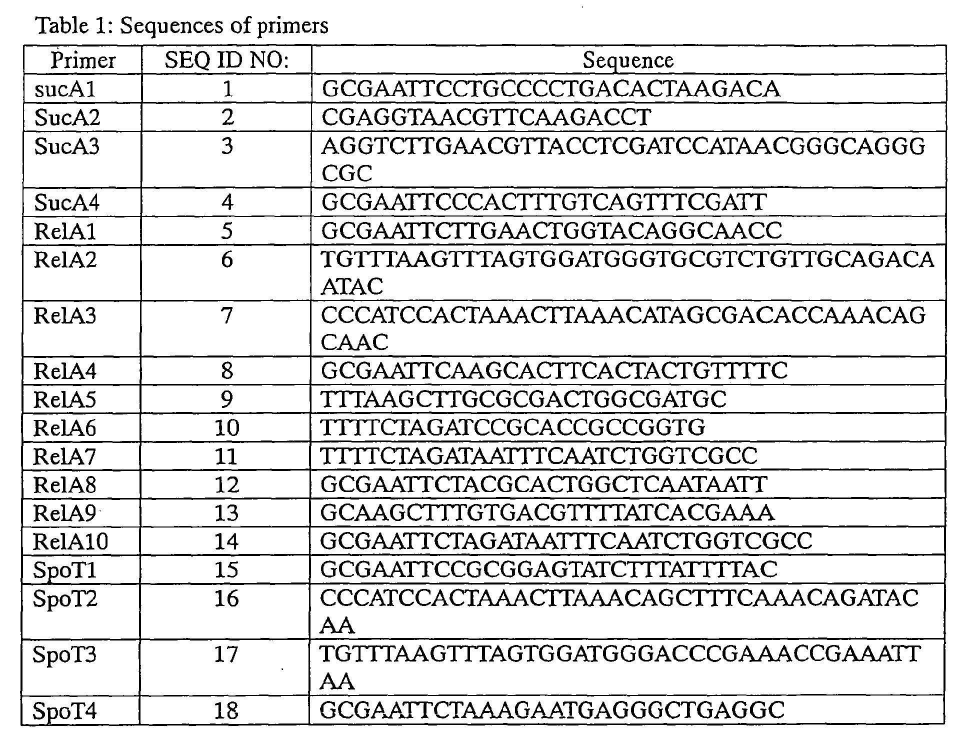

### Example 1

### <1> Acquisition of glutamic acid-overproducing strain of Escherichia coli

First, a *sucA* gene-disrupted strain of *Escherichia coli* wild-type strain was constructed in order to obtain glutamic acid-overproducing strains of *Escherichia coli.* A deletion type gene used for gene disruption was prepared by crossover PCR (see Link, A.J., Phillips, D., Church, G.M., J. Bacteriol., 179. pp.6228-6237, 1997). As primers, sucA1 to sucA4 were used. sucA1 and sucA4 were primers for amplifying the full length *sucA* gene and about 1000 bp each of flanking regions thereof at the both ends, and sucA2 and sucA3 constituted a primer set for deleting an internal partial sequence of ORF of the gene.

First PCR was performed using combinations of the primers sucA1 and sucA2 and the primers sucA3 and sucA4 and genomic DNA of an *Escherichia coli* wild-type strain MG1655 prepared by a usual method as a template. In this PCR, the primers sucA1 and sucA2 and the primers sucA4 and sucA3 were used in a molar ratio of 10:1. Second PCR was performed using the obtained product of the first PCR as a template and the primers sucA1 and sucA4. The *sucA* gene amplified by the second PCR had a deletion of an internal sequence of ORF. The both ends of the amplified DNA fragment were digested with the restriction enzyme *EcoRI*.

The plasmid pMAN997 having a temperature sensitive replication origin was digested with the same restriction enzyme *EcoRI*, purified and ligated to the aforementioned amplified fragment using DNA ligation Kit Ver. 2 (Takara Shuzo). *Escherichia coli* JM109 competent cells (Takara Shuzo) were transformed with the above ligation reaction mixture, inoculated on an LB agar plate containing 25 µg/ml of ampicillin (Ap, Sigma) (LB + Ap plate) and cultured at 30°C to select colonies. The colonies were cultured in the LB medium containing 25 µg/ml ofAp in test tubes at 30°C, and plasmids were extracted from the cells using Wizard Plus Miniprep (Promega). These plasmids were digested with *EcoRI,* and a plasmid containing a fragment of a target length was selected as a plasmid for gene disruption (pMANΔsucA).

A target host was transformed with pMANΔsucA, and colonies were selected on LB + Ap plates at 30°C. The selected colonies were cultured overnight at 30°C as liquid culture, diluted 10³ times and inoculated on LB + Ap plates, and colonies were selected at 42°C. The selected colonies were spread on LB + Ap plates and cultured at 30°C. Then, the cells corresponding to 1/8 of each plate were suspended in 2 ml of LB medium and cultured at 42°C for 4 to 5 hours with shaking. The culture broth diluted 10⁵ times was inoculated on an LB plate. Among the obtained colonies, several hundreds of colonies were inoculated on an LB plate and an LB + Ap plate, and their growth was checked to confirm Ap sensitivity or resistance. Colony PCR was performed for several Ap sensitive strains to confirm disruption of the *sucA* gene. Thus, MG1655ΔsucA was obtained.

### <2> Acquisition of relA gene-disrupted strain and spoT gene-disrupted strain from glutamic acid-overproducing strain of Escherichia coli

From MG1655ΔsucA obtained in <1>, strains were constructed in which the *relA* gene, the *spoT* gene or both of these genes were disrupted. Disruption of each gene was performed by crossover PCR. As primers for *relA* gene and *spoT* gene, relA1 to relA4 and spoT1 to spoT4 were used. relA1 and relA4, and spoT1 and spoT4 were primers for amplifying the full length *relA* gene and *spoT* gene and about 1000 bp each of flanking regions thereof at the both ends, respectively, and relA2 and relA3, and spoT2 and spoT3 constituted primer sets for deleting internal partial sequences of ORF of the genes, respectively.

First PCR was performed using combinations of the primers relA1 and relA2 and the primers relA3 and relA4 and genomic DNA of the *Escherichia coli* wild-type strain MG1655 prepared by a usual method as a template. In this PCR, the primers relA1 and relA2 and the primers relA4 and relA3 were used in a molar ratio of 10:1. Second PCR was performed using the obtained product of the first PCR as a template and the primers relA1 and relA4.

Furthermore, first PCR was also performed using combinations of the primers spoT1 and spoT2 and the primers spoT3 and spoT4 and genomic DNA of the *Escherichia coli* wild-type strain MG1655 prepared by a usual method as a template. In this PCR, the primers spoT1 and spoT2 and the primers spoT4 and spoT3 were used in a molar ratio of 10:1. Second PCR was performed using the obtained product of the first PCR as a template and the primers spot1 and spoT4.

The *relA* gene and *spoT* gene amplified by the second PCR each had a deletion of an internal sequence of ORF.

The plasmid pMAN997 having a temperature sensitive replication origin was digested with the same restriction enzyme *EcoRI,* purified and ligated to the aforementioned amplified fragment using DNA ligation Kit Ver. 2 (Takara Shuzo). *Escherichia coli* JM109 competent cells (Takara Shuzo) were transformed with the above ligation reaction mixture, inoculated on an LB agar plate containing 25 µg/ml of ampicillin (Ap, Sigma) (LB + Ap plate) and cultured at 30°C to select colonies. The colonies were cultured in the LB medium containing 25 µg/ml of Ap in test tubes at 30°C, and plasmids were extracted from the cells using Wizard Plus Miniprep (Promega). These plasmids were digested with *EcoRI,* and plasmids containing a fragment of a target length were selected as plasmids for gene disruption (pMANΔrelA and pMANΔspoA).

MG1655ΔsucA obtained in <1> was transformed with pMANΔrelA or pMAN spoA, and colonies were selected on LB + Ap plates at 30°C. The selected colonies were cultured overnight at 30°C as liquid culture, diluted 10³ times and inoculated on LB + Ap plates, and colonies were selected at 42°C. The selected colonies were spread on LB + Ap plates and cultured at 30°C. Then, the cells corresponding to 1/8 of each plate were suspended in 2 ml of LB medium and cultured at 42°C for 4 to 5 hours with shaking. The culture broth diluted 10⁵ times was inoculated on an LB plate. Among the obtained colonies, several hundreds of colonies were inoculated on an LB plate and an LB + Ap plate, and their growth was checked to confirm Ap susceptibility or resistance. Colony PCR was performed for several Ap susceptible strains to confirm disruption of the *sucA* gene. Thus, MG1655 sucA relA and MG1655 sucA spoT were obtained.

Furthermore, the *spoT* gene of MG1655ÄsucAÄrelA was disrupted in the same manner as described above to obtain MG1655ÄsucAÄrelAÄspoT.

Each gene-disrupted strain obtained as described above was evaluated for the glutamic acid-producing ability. The strains were cultured in a medium containing 40 g/L of glucose, 1 g/L of MgSO₄•7H₂O, 1 g/L of KH₂PO₄, 16 g/L of (NH₄)₂SO₄, 10 mg/L of FeSO₄• 7H₂O, 10 mg/L of MnSO₄• 4-5 H₂O, 2 g/L of yeast extract and 50 g/L of CaCO₃ contained in a 500-mL volume Sakaguchi flask. The volume of the culture broth at the start of the culture was 20 mL, and the culture was performed at 37°C for 24 hours with shaking by reciprocal movement at a rotation rate of 120 rpm. The medium, vessels and so forth were all subjected to autoclave sterilization before use. The cell density, glucose concentration and glutamic acid accumulation amount in the culture broth were measured. The cell density was determined by measuring turbidity at 562 nm of the culture broth diluted with 0.1 N hydrochloric acid to a suitable concentration using a spectrophotometer (Beckman). The residual glucose concentration and glutamic acid concentration were measured using Biotech Analyzer (Sakura Seiki) for the culture supernatant diluted with water to a suitable concentration after removal of the cells by centrifugation. The results are shown in Table 2.

**Table 2:**

| Glutamic acid-producing ability of various glutamic acid-producing bacteria | | | | |
|---|---|---|---|---|
| Strains | MG1655 ΔsucA | MG1655 ΔsucA ΔrelA | MG1655 ΔsucAΔrelA ΔspoT | MG1655 ΔsucA ΔspoT |
| OD₅₆₂ | 16.6 | 10.8 | 8.6 | 15.7 |
| Glucose (g/L) | 0.0 | 20.2 | 22.6 | 0.0 |
| Glutamic acid(g/L) | 13.8 | 3.6 | 3.2 | 15.7 |
| Yield | 34.5% | 18.1% | 18.4% | 39.3% |

As shown in Table 2, it was confirmed that all of the growth, sugar consumption and glutamic acid yield were markedly reduced in the *relA*-disrupted strains. On the other hand, any effect was not substantially observed due to the deficiency of the *spoT* gene.

### Example 2: Construction of ppGpp overproducing plasmid

A strain overproducing ppGpp was constructed by amplifying the total region of the *relA* gene or a region encoding the catalytic domain of the *relA* gene product (*relA* *). As primers for amplification, four kinds of different plasmids (pMrelA, pMrelA*, pSTVrelA, pSTVrelA*) were constructed.

### <1> Construction of pMrelA and pMrelA*

Plasmids pMrelA and pMrelA* were constructed as follows.

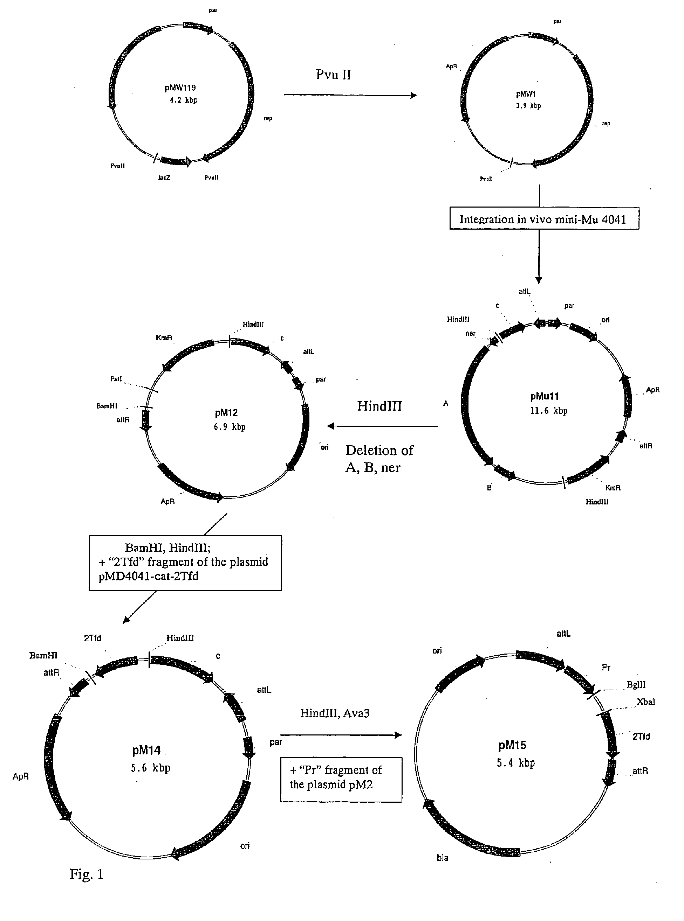

The plasmid pMW119 (Nippon Gene) was digested with the restriction enzyme *PvuII* and self-cyclized to obtain a plasmid pMW1. Then, the mini-Mud 4041 vector (Miller, "A short course in bacterial genetics", Cold Springs Harbor Press (1992) 385-400) was incorporated into the plasmid pMW1 in a conventional manner to obtain a plasmid pMu11. pMu11 was digested with the restriction enzyme *HindIII* and then self-circulated to obtain a plasmid pM12 from which the genes A and B encoding the transposase derived from Mu phage and the *ner* gene encoding a negative control factor were removed. Then, the plasmid pM12 was digested with the restriction enzymes *BamHI* and *HindIII* and ligeted to a region containing the *ter* and *fd* regions (2Tfd) excised from the plasmid pMD4041-cat-2Tfd by digestion with the restriction enzymes *BamHI* and *HindIII* to obtain a plasmid pM14 (Fig. 1).

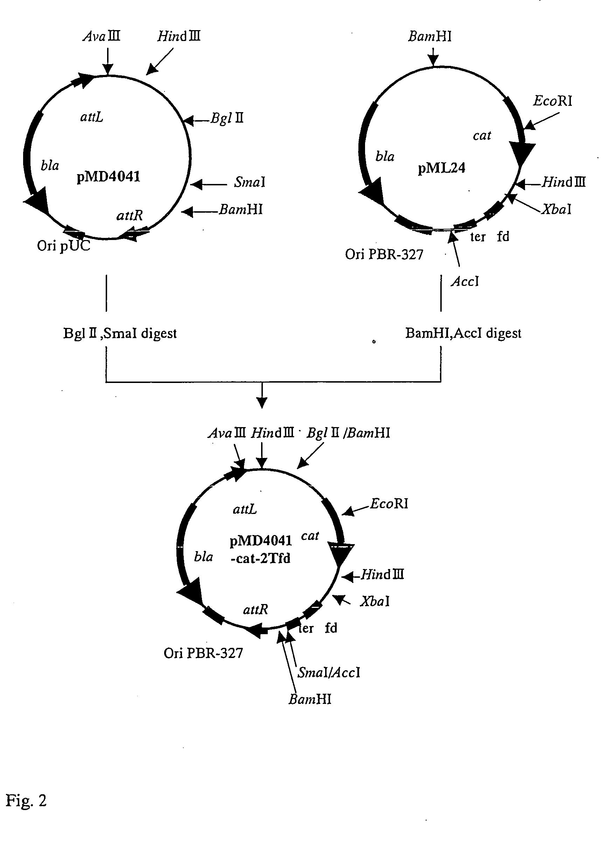

The aforementioned plasmid pMD4041-cat-2Tfd was obtained as follows. The plasmid pML24 (Trukhan et al., Biotechnologiya (in Russian) 4, No.3 (1988), 325-334; European Patent Application Laid-open No. 1234883) was digested with the restriction enzymes *BamHI* and *AccI* and blunt-ended with T4 DNA polymerase. To this product, the plasmid pMD4041 digested with *BglII* and *SmaI* and blunt-ended with DNA polymerase was ligated to obtain a plasmid pMD4041-cat-2Tfd (Fig. 2). pMD4041 is a plasmid obtained by digesting pMu4041 (mini-Mud 4041, Faelen, M., Useful Mu and mini-Mu derivatives, In: Phage Mu, Symonds et al., eds., Cold Spring Harbor Laboratory, New York, 1987, pp.309-316) with *HindIII* to excise the genes A and B encoding the transposase of Mu phage and the *ner* gene encoding a negative control factor and re-cyclizing it (European Patent Application Laid-open No. 1149911).

The plasmid pM14 was digested with the restriction enzymes *AvaIII* and *HindIII* and ligeted to a fragment excised from the plasmid pM2 (Japanese Patent Laid-open No. 2001-346578, European Patent Application Laid-open No. 1149911) by digestion with the restriction enzymes *AvaIII* and *BglII* and containing the P_{R} promoter derived from λ phage. Thus, a plasmid pM15 was obtained (Fig. 1).

The plasmid pM15 was digested with the restriction enzymes *HindIII* and *XbaI* to obtain a vector fragment. Furthermore, PCR was performed using a genomic DNA of the *Escherichia coli* wild-type strain MG1655 as a template and the primers relA5 and relA6, and the amplification product was digested with *HindIII* and *XbaI* to obtain a DNA fragment containing the *relA* gene. This DNA fragment and the aforementioned vector fragment (pM15) were ligated using DNA Ligation Kit Ver. 2 (Takara Shuzo). Thus, a plasmid pMrelA was obtained.

Separately, PCR was performed using a genomic DNA of MG1655 as a template and the primers relA5 and relA7, and the amplification product was digested with *HindIII* and *XbaI* to obtain a DNA fragment containing the *relA** gene. This DNA fragment and the aforementioned vector fragment (pM15) were ligated using DNA Ligation Kit Ver. 2 (Takara Shuzo). Thus, a plasmid pMrelA* was obtained.

### <2> Construction of pSTVrelA and pSTVrelA*

Plasmids pSTVrelA and pSTVrelA* were constructed as follows.

The plasmid pSTV29 (Takara Shuzo) was digested with the restriction enzymes *EcoRI* and *HindIII* to obtain a vector fragment. Furthermore, PCR was performed using a genomic DNA of the *Escherichia coli* wild-type strain MG1655 and the primers relA8 and relA9, and the amplification product was digested with the restriction enzymes *EcoRI* and *HindIII* to obtain a DNA fragment containing the *relA* gene. These DNA fragments were ligeted to obtain a plasmid pSTVrelA.

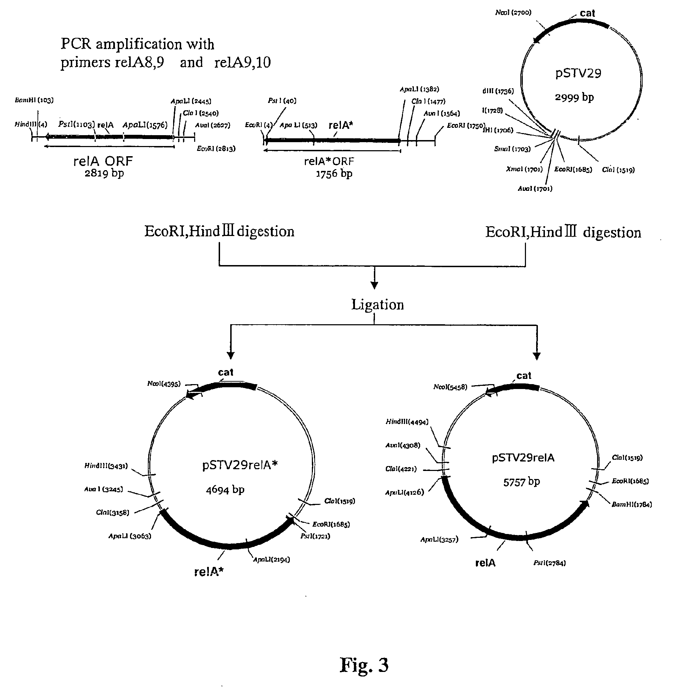

Separately, PCR was performed using a genomic DNA of MG1655 as a template and the primers relA9 and relA10, and the amplification product was digested with *EcoRI* and *HindIII* to obtain a DNA fragment containing the *relA** gene. This DNA fragment and the aforementioned vector fragment (pSTV29) were ligated to obtain a plasmid pSTVrelA* (Fig. 3).

### Example 3: Introduction of relA gene into glutamic acid-overproducing strain of Escherichia coli and relA gene-disrupted strain thereof and effect thereof on glutamic acid production

The plasmids pM15, pMrelA and pMrelA* obtained in Example 2 were used to transform MG1655ΔsucA and MG1655ΔsucAΔrelA obtained in Example 1.

Each transformant was evaluated for the glutamic acid production ability. The strains were cultured in a medium containing 40 g/L of glucose, 1 g/L of MgSO₄•7H₂O, 1 g/L of KH₂PO₄, 16 g/L of (NH₄)₂SO₄, 10 mg/L of FeSO₄•7H₂O, 10 mg/L of MnSO₄• 4-5 H₂O, 2 g/L of yeast extract, 50 g/L of CaCO₃ and 100 µg/mL of ampicillin contained in a 500-mL volume Sakaguchi flask. The volume of the culture broth at the start of the culture was 20 mL, and the culture was performed at 37°C for 24 hours with shaking by reciprocal movement at a rotation rate of 120 rpm. The medium, vessels and so forth were all subjected to autoclave sterilization before use. The cell density, glucose concentration and glutamic acid accumulation amount in the culture broth were measured. The cell density was determined by measuring turbidity at 600 nm of the culture broth diluted with 0.1 N hydrochloric acid to a suitable concentration using a spectrophotometer (Beckman). The residual glucose concentration and glutamic acid concentration were measured using Biotech Analyzer (Sakura Seiki) for the culture supernatant diluted with water to a suitable concentration after removal of the cells by centrifugation. The results are shown in Table 3.

**Table 3:**

| Glutamic acid-producing ability of various glutamic acid-producing bacteria. | | | | | | |
|---|---|---|---|---|---|---|
| Strain | MG1655 ΔsucA /pM15 | MG1655 ΔsucA /pMrelA* | MG1655 ΔsucA /pMrelA | MG1655 ΔsucAΔrelA /pM15 | MG1655 ΔsucAΔrelA /pMrelA* | MG1655 ΔsucAΔrelA /pMrelA |
| OD₅₆₂ | 17.8 | 17.9 | 17.5 | 7.3 | 7.2 | 16.9 |
| Glucose (g/L) | 0.0 | 0.0 | 0.0 | 21.3 | 23.2 | 0.0 |
| Glutamic acid (g/L) | 15.8 | 16.7 | 19.2 | 1.7 | 2.7 | 18.5 |
| Yield | 39.5% | 41.7% | 48.1% | 9.1% | 16.1% | 47.7% |

In the pMrelA*-introduced strains, improvement of the glutamic acid yield was observed, whereas effect on recovery of growth of the *relA*-disrupted strain was not substantially observed. On the other hand, in the pMrelA-introduced strain, growth of the *relA*-deficient strain was completely recovered with improvement of the glutamic acid yield, and the glutamic acid yield was markedly improved compared with the control (MG1655ΔsucA/pM15). That is, it was confirmed that the glutamic acid yield was improved due to existence of multiple copies of the *relA* gene.

### Example 4: Introduction of relA gene and relA * gene into threonine-overproducing strain of Escherichia coli and effect thereof on threonine production

The plasmids pM15, pMrelA and pMrelA* obtained in Example 2 were used to transform the *Escherichia coli* threonine-producing strain VKPM B-3996 (Japanese Patent No. 2775948).

Each strain was cultured in a medium containing 40 g/L of glucose, 1 g/L of MgSO₄• 7H₂O, 1 g/Lof KH₂PO₄, 16 g/Lof (NH₄)₂SO₄, 10 mg/L of FeSO₄• 7H₂O, 10 mg/L of MnSO₄• 4-5 H₂O, 2 g/L of yeast extract, 50 g/L of CaCO₃ and 100 µg/mL of ampicillin contained in a 500-mL volume Sakaguchi flask. The volume of the culture broth at the start of the culture was 20 mL, and the culture was performed at 37°C for 24 hours with shaking by reciprocal movement at a rotation rate of 120 rpm. The medium, vessels and so forth were all subjected to autoclave sterilization before use. The cell density and glucose concentration in the culture broth were measured. The cell density was determined by measuring turbidity at 600 nm of the culture broth diluted with 0.1 N hydrochloric acid to a suitable concentration using a spectrophotometer (Beckman). The residual glucose concentration was measured using Biotech Analyzer (Sakura Seiki) for the culture supernatant diluted with water to a suitable concentration after removal of the cells by centrifugation. The threonine concentration was measured using an amino acid analyzer L-8500 (Hitachi) for the culture supernatant diluted with 0.02 N hydrochloric acid to a suitable concentration after removal of the cells by centrifugation. The results are shown in Table 4.

**Table 4:**

| Threonine-producing ability of various threonine-producing bacteria | | | | |
|---|---|---|---|---|
| Strain | OD₅₆₂ | Threonine accumulation (g/L) | Glucose concentration (g/L) | Threonine yield |
| B-3996/pM15 | 15.47 | 10.99 | 0.0 | 25.62% |
| B-3996/pMrelA | 16.21 | 12.17 | 0.0 | 29.60% |
| B-3996/pMrelA* | 16.21 | 11.28 | 0.0 | 26.29% |

About 1% of improvement of the threonine yield was observed for the pMrelA*-introduced strain, whereas about 4% of improvement of the threonine yield was observed for the pMrelA-introduced strain. That is, it was confirmed that the threonine yield was improved because of existence of multiple copies of the *relA* * gene or *relA* gene.

### Example 5: Introduction of relA gene and relA* gene into isoleucine-overproducing strain of Escherichia coli and effect thereof on isoleucine production

The plasmids pSTV29, pSTVrelA and pSTVrelA* obtained in Example 2 were used to transform the *Escherichia coli* isoleucine-producing strain B-3996/pMWD5 (Japanese Patent Laid-Open No. 08-047397, U.S. Patent No. 5,998,178).

Each strain was cultured in a medium containing 40 g/L of glucose, 1 g/L of MgSO₄• 7H₂O, 1 g/L of KH₂PO₄, 16 g/L of (NH₄)₂SO₄, 10 mg/L of FeSO₄• 7H₂O, 10 mg/L of MnSO₄• 4-5 H₂O, 2 g/L of yeast extract, 50 g/L of CaCO₃, 100 µg/mL of ampicillin and 25 µg/mL of chloramphenicol contained in a 500-mL volume Sakaguchi flask. The volume of the culture broth at the start of the culture was 20 mL, and the culture was performed at 37°C for 24 hours with shaking by reciprocal movement at a rotation rate of 120 rpm. The medium, vessels and so forth were all subjected to autoclave sterilization before use. The cell density and glucose concentration in the culture broth were measured. The cell density was determined by measuring turbidity at 600 nm of the culture broth diluted with 0.1 N hydrochloric acid to a suitable concentration using a spectrophotometer (Beckman). The residual glucose concentration was measured using Biotech Analyzer (Sakura Seiki) for the culture supernatant diluted with water to a suitable concentration after removal of the cells by centrifugation. The isoleucine concentration was measured using an amino acid analyzer L-8500 (Hitachi) for the culture supernatant diluted with 0.02 N hydrochloric acid to a suitable concentration after removal of the cells by centrifugation. The results are shown in Table 5.

**Table 5:**

| Isoleucine-producing ability of various isoleucine-producing bacteria | | | | | | | |
|---|---|---|---|---|---|---|---|
| Strain | OD₅₆₂ | | Isoleucine accumulation (g/L) | | Glucose concentration (g/L) | | Isoleucine yield |
| | 24 hours | 31 hours | 24 hours | 31 hours | 24 hours | 31 hours | |
| B-3996/pMWD5, pSTV29 | 15.65 | 14.41 | 6.42 | 8.16 | 4.3 | 0.3 | 19.2% |
| B-3996/pMWD5, pSTVrelA | 11.16 | 14.61 | 3.79 | 8.93 | 25.1 | 1.0 | 21.3% |
| B-3996/pMWD5, PSTVrelA* | 17.97 | 15.77 | 9.23 | 9.46 | 0.7 | 0.4 | 22.3% |

About 3% of improvement of the isoleucine yield was observed for the pSTVrelA*-introduced strain, whereas about 2% of improvement of the isoleucine yield was observed for the pSTVre1A-introduced strain. That is, it was confirmed that the isoleucine yield was improved because of existence of multiple copies of the *relA** gene or *relA* gene.

### Example 6: Introduction of relA gene and relA* gene into lysine-overproducing strain of Escherichia coli and effect thereof on lysine production

As an L-lysine-producing strain of *Escherichia coli,* the WC196 strain was used. This strain was bred by imparting AEC resistance to the W3110 strain derived from *Escherichia coli* K-12. This strain was designated as the *Escherichia coli* AJ13069 strain, and was deposited at the National Institute of Bioscience and Human-Technology, Agency of Industrial Science and Technology (presently, the independent administrative agency, International Patent Organism Depositary, National Institute of Advanced Industrial Science and Technology, postal code: 305-8566, Chuo Dai-6, 1-1 Higashi 1-Chome, Tsukuba-shi, Ibaraki-ken, Japan on December 6, 1994 and received an accession number of FERM P-14690. Then, the deposit was converted to an international deposit under the provisions of the Budapest Treaty on September 29, 1995, and received an accession number of PERM BP-5252 (see International Patent Publication WO96/17930). Furthermore, pCAB1 was used as a plasmid for producing a lysine-overproducing strain. This plasmid carried a mutant *lysC* gene encoding aspartokinase for which feedback inhibition by L-lysine was desensitized, mutant *dapA* gene encoding dihydrodipicolinate synthase for which feedback inhibition by L-lysine was desensitized and *dapB* gene encoding dihydrodipicolinate reductase (Japanese Patent Laid-open No. 11-192088, U.S. Patent No. 6,040,160). The *Escherichia coli* lysine-producing strain WC196 was transformed with this plasmid to obtain a lysine-overproducing strain WC196/pCAB1.

Furthermore, plasmids pM15 and pMrelA obtained in Example 2 were used to transform the *Escherichia coli* lysine-producing strain WC196/pCAB1 and thereby obtain WC196/pCAB1/pM15 and WC196/pCAB1/pMrelA.

Each strain was cultured in a medium containing 40 g/L of glucose, 1 g/L of MgSO₄• 7H₂O, 1 g/L of KH₂PO₄, 16 g/L of (NH₄)₂SO₄, 10 mg/L of FeSO₄• 7H₂O, 10 mg/L of MnSO₄• 4-5 H₂O, 2 g/L of yeast extract, 50 g/L of CaCO₃, 100 µg/mL of ampicillin and 100 µg/mL of streptomycin contained in a 500-mL volume Sakaguchi flask. The volume of the culture broth at the start of the culture was 20 mL, and the culture was performed at 37°C for 42 hours with shaking by reciprocal movement at a rotation rate of 120 rpm. The medium, vessels and so forth were all subjected to autoclave sterilization before use. The cell density and glucose concentration in the culture broth were measured. The cell density was determined by measuring turbidity at 600 nm of the culture broth diluted with 0.1 N hydrochloric acid to a suitable concentration using a spectrophotometer (Beckman). The residual glucose concentration was measured using Biotech Analyzer (Sakura Seiki) for the culture supernatant diluted with water to a suitable concentration after removal of the cells by centrifugation. The lysine concentration was measured using Biotech Analyzer (Sakura Seiki) for the culture supernatant diluted with water to a suitable concentration after removal of the cells by centrifugation. The results obtained when all of the glucose in the medium was consumed (culture time: 42 hours) are shown in Table 6.

**Table 6:**

| Lysine-producing ability of various lysine-producing bacteria (results obtained after 42 hours of culture) | | | |
|---|---|---|---|
| Strain | OD₅₆₂ | Lysine accumulation | Lysine yield |
| WC196/pCAB1/pM15 | 12.49 (0.037) | 14.7 (1.10) | 36.69% (2.74) |
| WC196/pCAB1/pMre1A | 14.39 (1.66) | 15.8 (0.17) | 39.56% (0.44) |

The numerical values in the parentheses represent standard deviations when n is 3.

While the invention has been described in detail with reference to preferred embodiments thereof, it will be apparent to one skilled in the art that various changes can be made, and equivalents employed, without departing from the scope of the invention. Each of the aforementioned documents is incorporated by reference herein in its entirety, including the foreign priority document, JP2003-166654.

## Claims

1. A method for producing an L-amino acid comprising
a) culturing a bacterium having an ability to produce an L-amino acid in a medium to allow accumulation of the L-amino acid in a culture, and
b) collecting the L-amino acid from the culture, wherein the bacterium is modified so that an activity to synthesize ppGpp is increased.

2. The method according to claim 1, wherein the bacterium is modified so that an activity of an enzyme synthesizing ppGpp is increased.

3. The method according to claim 2, wherein said enzyme is a RelA protein or a catalytic domain of the RelA protein.

4. The method according to claim 3, wherein the activity of the RelA protein is increased by
a) increasing the copy number of a *relA* gene or a partial region of the *relA* gene encoding a catalytic domain of the RelA protein, or
b) modifying an expression regulatory sequence of the relA gene so that intracellular expression of the *relA* gene or a partial region of the *relA* gene encoding a catalytic domain of the RelA protein of the bacterium is enhanced.

5. The method according to claim 3, wherein the RelA protein is a protein defined in the following (A) or (B):
(A) a protein which has the amino acid sequence of SEQ ID NO: 20.
(B) a protein which has the amino acid sequence of SEQ ID NO: 20 including substitution, deletion, insertion or addition of one or several amino acid residues,
and wherein said protein has an activity to synthesize ppGpp.

6. The method according to claim 3, wherein the RelA protein is encoded by a DNA defined in the following (a) or (b):
(a) a DNA has the nucleotide sequence of SEQ ID NO: 19,
(b) a DNA which is hybridizable with the nucleotide sequence of SEQ ID NO: 19 under stringent conditions,
and wherein said DNA encodes a protein which has an acitivity to syntehsize ppGpp.

7. The method according to any one of claims 1 to 6, wherein the L-amino acid is selected from the group consisting of L-glutamic acid, L-threonine, L-isoleucine, L-lysine, L-histidine, L-valine, L-arginine, L-leucine, L-phenylalanine and L-triptophan.

8. The method according to claim 7, wherein the L-amino acid is selected from the group consisting of L-glutamic acid, L-threonine, L-isoleucine or L-lysine.

9. The method according to claim 7, wherein the bacterium is a bacterium belonging to the genus *Escherichia.*

10. A bacterium which has an ability to produce an L-amino acid and which is modified so that an activity of intracellular RelA protein is increased.
